## Europäisches Patentamt

**European Patent Office**

**Office européen des brevets**

(11) Numéro de publication: **0 170 584**

**B1**

(12) **FASCICULE DE BREVET EUROPEEN**

(45) Date de publication du fascicule du brevet: **14.03.90**

(51) Int. Cl.⁵: **C 12 Q 1/68, C 12 Q 1/10**

(21) Numéro de dépôt: **85401435.4**

(22) Date de dépôt: **12.07.85**

(54) **Sonde d'ADN et procédé pour la détection de "Shigelles" et des souches entéro-invasives de Escherichia coli.**

(30) Priorité: **13.07.84 FR 8411187**

(43) Date de publication de la demande:
**05.02.86 Bulletin 86/06**

(45) Mention de la délivrance du brevet:
**14.03.90 Bulletin 90/11**

(84) Etats contractants désignés:
**AT BE CH DE GB IT LI LU NL SE**

(56) Documents cités:
**CHEMICAL ABSTRACTS, vol. 98, no. 19, 9 mai 1983, page 88, no. 155688c, Columbus, Ohio, US; T.L. HALE et al.: "Characterization of virulence plasmids and plasmid-associated outer membrane proteins in Shigella flexneri, Shigella sonnei, and Escherichia coli", & INFECT. IMMUN. 1983, 40(1), 340-50**

(73) Titulaire: **INSTITUT PASTEUR**
**25-28, rue du Docteur Roux**
**F-75724 Paris Cédex 15 (FR)**

(73) Titulaire: **INSTITUT NATIONAL DE LA SANTE ET DE LA RECHERCHE MEDICALE (INSERM)**
**101, rue de Tolbiac**
**F-75654 Paris Cédex 13 (FR)**

(72) Inventeur: **Sansonetti, Philippe**
**131 Bld. Brune**
**F-75014 Paris (FR)**
Inventeur: **Boileau, Catherine**
**67, rue de Courcelles**
**F-75008 Paris (FR)**
Inventeur: **D'Hauteville, Hélène**
**10, rue des Volontaires**
**F-75015 Paris (FR)**

(74) Mandataire: **Gutmann, Ernest et al**
**S.C. Ernest Gutmann - Yves Plasseraud 67, boulevard Haussmann**
**F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 170 584 B1

(56) Documents cités:

CHEMICAL ABSTRACTS, vol. 99, no. 25, 19 décembre 1983, page 171, no. 207154t, Columbus, Ohio, US; P.J. SANSONETTI et al.: "Molecular comparison of virulence plasmids in Shigella and enteroinvasive Escherichia coli", & ANN. MICROBIOL. 1983, 134A(3), 295-318

BIOLOGICAL ABSTRACTS, vol. 28, 1984, Philadelphia, US; P. SANSONETTI et al.: "Part of plasmids in the invasive capacity of Shigella and Escherichia-coli", & COLLOQUE INSERM 183, pages 537-548

CHEMICAL ABSTRACTS, vol. 96, no. 19, 10 mai 1982, page 440, no. 159173n, Columbus, Ohio, US; P.J. SANSONETTI et al.: "Involvement of a plasmid in the invasive ability of Shigella flexneri", & INFECT. IMMUN. 1982, 35(3), 852-60

CHEMICAL ABSTRACTS, vol. 100, no. 1, January 2, 1984, page 116, no. 1322b, Columbus, Ohio, US; Y. BAO et al.: "Studies on drug-resistance variation of Shigella. II. Detection of plasmid DNAs from multiple drug-resistance Shigella" & ZHONGHUA WEISHENGWUXUE HE MIANYIXUE ZAZHI 1983, 3(4), 257-60

**Description**

L'invention concerne un procédé et des moyens, plus particulièrement une sonde d'ADN, pour la détection des shigelles et des souches entéro-invasives de E. coli. Elle concerne plus particulièrement, car c'est dans ce cas que leur application semble présenter le plus d'intérêt, des moyens permettant le diagnostic in vitro des syndromes de dysenteries ou de diarrhées dues à des bactéries du genre en question, dont est connue la capacité d'envahir les cellules de la muqueuse du colon et d'engendrer des dommages tissulaires. D'une façon générale ces affections seront ci-après dénommées Shigelloses, que celles-ci soient dues à des shigelles ou encore à des souches entéro-invasives de E. coli, lesquelles sont néanmoins connues pour produire une dysenterie du type de celles engendrées par les shigelles. Il a d'ailleurs été constaté que ces souches de E. coli appartiennent à divers sérotypes O, lesquels donnent souvent lieu à des réactions croisées avec les séro-groupes des shigelles A, B et C.

Les shigelloses sont endémiques dans le monde entier. Elles représentent cependant un problème de santé publique particulièrement grave dans les régions tropicales et les pays en développement, dans lesquels dominent *Shigella Dysenteriae* et *S. flexneri*. Dans les pays industrialisés l'agent étiologique principal est *S. sonnei*, bien que l'on rencontre des cas sporadiques de shigellose dûs à *S. flexneri*, *S. boydii* et au susdit sérotype entéroinvasif *E. coli*.

La sévérité des syndromes dysentériques implique des diagnostics rapides et la mise en oeuvre dans délai de traitements de la maladie. La détection de ces espèces bactériennes doit en outre reposer sur des procédés rapides, simples et de coût réduit. Malheureusement aucun procédé de ce type n'était disponible à l'heure actuelle. Les procédés d'identification des espèces bactériennes responsables impliquent des tests de virulence bactériologiques ou immunologiques relativement compliqués. L'un de ces test consiste dans l'induction d'une kérato-conjonctivite chez le cobaye par les bactéries virulents (Sereny, B. Acta Microbiol. Hung. [1957], *4*, 367—376). Un autre test quelquefois mis en oeuvre implique la réalisation in vitro de colonisation par ces bactéries de cultures en monocouches de cellulés humaines HeLa selon la technique décrite par Hale, T. L. et Formal, F. D., Infect. Immun. 1981, 32, 137—144. On conçoit que ces techniques ne peuvent être mises en oeuvre à grande échelle, à des fins de prévention ou de thérapie.

L'invention consiste dans l'aboutissement de recherches qui a permis l'isolement de sondes géniques permettant le diagnostic grâce à des hybridations sélectives avec les acides nucléiques préalablement isolés ou rendus accessibles d'isolats bactériens, et ce quelle que soit l'espèce bactérienne particulière des shigelles ou E. coli entéro-invasives responsables de l'affection.

Il est connu que toutes les souches virulentes de shigelles ou de *E. coli* entéro-invasifs hébergent un plasmide de haut poids moléculaire (120—140 mégadaltons) (Mdal), plasmide qui s'est avéré indispensable à une étape fondamentale du pouvoir pathogène de ces bactéries, à savoir la capacité de pénétrer dans les cellules épithéliales. En particulier P. J. Sansonetti et coll (Infection and Immunity, 1982, vol. 35, *3*, 852—860) ont démontré que la virulence d'une souche M 90 T (sérotype 5) était liée à la présence d'un plasmide de 140-Mdal. Après marquage de ce plasmide (pWR100) par un transposon Tn5 ayant un facteur de résistance à la kanamycine (plasmide pWR 110) ces auteurs devaient montrer que la perte par les souches virulentes de *S. flexneri* de ce plasmide, par exemple par incubation de cultures de ces souches à 42°C, entraînaient également la perte de la virulence. La production de délétions importantes du plasmide entraînait encore les mêmes effets.

Dans un article postérieur P. J. Sansonetti et coll (Ann. Microbiol. de l'Institut Pasteur 1983 134 A, 295—318) établissaient définitivement l'intervention d'un plasmide de ce type, autrement dit d'un élément extra-chromosomique, dans la virulence de toutes les souches entéro-invasives appartenant au genre *Shigella* et *Escherichia coli*. Cependant, si les expériences d'hybridation croisée entre les plasmides obtenus à partir de différentes espèces de *Shigella* ou de *E. coli* montraient des séquences homologues réparties sur l'ensemble de ces plasmides, il était également constaté que d'une espèce à l'autre les profils de coupures par les endo-nucléases de restriction, notamment Bam HI, EcoRI, etc, ne montraient pas de parenté évidente. De plus ces auteurs devaient constater que le plasmide de 140 Mdal[=140×106 daltons] qu'ils avaient utilisé à titre de sonde pour leurs études (plasmides de virulence de E. coli entéro invasif 4.608-58) hybridait non seulement avec les plasmides de virulence des autres souches, mais encore avec d'autres plasmides, notamment des plasmides plus petits présents dans des souches aussi bien virulentes que non virulentes. Ces constatations allaient donc a priori à l'encontre de celles qui ont été faites dans le cadre de la présente invention, avec des séquences particulières isolées à partir du plasmide de 140 Mdal de la souche M 90T de *S. flexneri* (sérotype 5), séquences qui se sont révélées présenter une sélectivité telle, que leur mise en oeuvre permet la discrimination avec une sécurité proche de 100%, sinon totale, de shigelles ou souches entéro-invasives de *E. coli* virulentes dans des isolats bactériens, notamment obtenus à partir de selles diarrhéiques.

La sonde selon l'invention pour la détection des shigelles et des *E. coli* entéro invasifs est caractérisée en ce qu'elle comprend une séquence d'acide nucléique originaire du plasmide de virulence de 140 Mdal. de la souche M 90 T de *Shigella flexneri*, ayant une taille au plus égale à environ 27 kb et comprenant la totalité ou une partie du fragment Bam HI de 27 kb qui peut être isolé à partir du susdit plasmide de virulence.

Des sondes particulièrement préférées sont constituées par des séquences EcoRI ayant respectivement des tailles de l'ordre 7,6 kb, 11,5 kb et 17 kb, ces séquences ayant au moins une partie en commun avec le susdit fragment Bam HI de 27 kb. Ces poids moléculaires apparents ont été déterminés par rapport à une droite de référence établie à l'aide des fragments de restriction obtenus par action d'EcoRI et de Hind III sur le phage λ en tant que moyens de référence.

Les fragments de référence et les fragments de la présente invention ont été séparés sur un gel d'agarose à 0,7% de électrophorèse en tampon E (Kado et Liu, Journal of Bacteriology, *1981* Vol. 145 pp. 1365—1373).

Il est entendu que dans ce qui précède "fragment Bam Hi" de 27 kb signifie le fragment qui peut être isolé à partir du plasmide de 140 Mdal. isolable (notamment par la méthode de Casse F. et coll. J. Gen Microbiol. (1979) 113:229—242) à partir de la susdite souche M 90 T de *S. flexneri,* par traitement de ce plasmide avec l'enzyme de restriction Bam HI et isolément parmi ces fragments de celui qui présente une taille de l'ordre de 27 kb. De même l'expression "fragments EcoRI" fait référence aux fragments qui peuvent être obtenus à partir d'un plasmide plus grand, par fragmentation de celui-ci par l'enzyme de restriction EcoRI et récupération ultérieure desdits fragments de restriction.

Il est encore entendu que dans les définitions qui précèdent l'indication que les sondes de l'invention comprennent "une partie du fragment Bam HI de 27 kb" fait référence au fait que ces sondes possèdent une partie en commun avec ce fragment Bam HI ou encore possédaient une telle partie en commun, dans la mesure où les délétions ultérieurement opérées dans les fragments en question auraient eu pour effet d'éliminer ces parties communes. Ces définitions s'étendent naturellement plus particulièrement aux fragments EcoRI (intacts) qui, avant les susdites délétions, comportaient une partie proche de l'une ou de l'autre des extrémités Bam HI dudit fragment Bam HI de 27 kb.

Les définitions qui précèdent s'étendent également encore à toute séquence d'ADN obtenue à partir d'un plasmide de virulence obtenu à partir d'une autre espèce de shigelles ou d'un quelconque *E. coli* entéro-invasif, dans la mesure où ces séquences donnent lieu à une hybridation sélective avec les séquences préférées obtenues à partir du plasmide 140 Mdal. de la souche M 90 T de *S. flexneri.*

Pour les études qui ont conduit à l'invention, la souche M 90 T de *S. flexneri* sérotype 5 (déposée à la collection nationale des cultures de microorganismes de l'Institut Pasteur CNCM le 6.06. 1984 ous le No. I-308) contenant un plasmide de virulence pWR 100 de 140 Mdal. a été utilisée. Cette souche présente les caractéristiques suivantes. Elle est lactose⁻, Kmˢ (sensible à la kanamycine) et vir⁺ (virulente).

On a eu recours à l'insertion *in vivo* du transposon Tn5 possédant un facteur de résistance Kmʳ, dans le plasmide pWR 100.

Pour ce faire, on a d'abord transféré par conjugaison bactérienne, dans une souche virulente de M 90 T, le plasmide F′ₜₛ lac 114: Tn5 issu de la souche de E. coli C 600 MU 735 déposée à la CNCM sous le No. I-307, codant pour le caractère lactose et pour la résistance à la kanamycine du fait de la présence du Tn5. Ce plasmide a une replication thermo-sensible et peut être perdu par culture à 42°C après l'évènement de transposition.

L'incorporation du plasmide F′ₜₛ lac 114:Tn5 dans la souche M 90 T a pour effet de conférer à celle-ci les caractères suivantes:

$$Lactose^+, Km^r, vir^+$$

La transposition de Tn5 à l'intérieur du plasmide pWR 100 dans une séquence génique non essentielle à l'expression de la virulence de la souche M 90 T, ne se manifeste pas par une modification du phénotype des bactéries en cause; mais ces bactéries transformées perdent le plasmide F′ₜₛ lac 114:Tn5 par incubation à 42°C.

Après la perte de ce plasmide, on peut isoler—parmi les souches Kmʳ qui ont retenu le plasmide pWR 100 (intact ou non délété)—deux catégories de souches présentant les phénotypes:

$$lactose^-, Km^r, vir^+$$
$$lactose^-, Km^r, vir^-$$

selon que le transposon a été incorporé dans un site non essentiel ou essentiel à la manifestation de la virulence.

La présence du transposon Tn5 dans le plasmide ou dans les plasmides PWR 100::Tn5 est repérée par hybridation en utilisant pour sonde une séquence marquée radioactivement de 3.200 paires de bases appartenant à Tn5.

Sur une banque de 1.000 clones testés, 12 seulement ont semblé répondre à l'ensemble de ces exigences. Les plasmides modifiés pWR 100::Tn5 ont été isolés à partir des souches lactose⁻, Kmʳ et vir⁻ et fragmentées par l'enzyme de restriction EcoRI.

Sur la base de la restriction par EcoRI, le Tn5 inactivant la virulence s'est avéré être systématiquement inséré dans l'un des trois fragments suivants (il existe 19 fragments EcoRI de pWR 100):

−7,6 kb
−11,5 kb
−17 kb.

Ceci a été confirmé par modification de la taille de ces fragments et hybridation avec la sonde Tn5 déjà mentionnée. On a alors cloné des fragments EcoRI correspondants, isolés à partir d'un même plasmide par restriction EcoRI, dans le site EcoRI de pBR 325 préparé à partir de la souche E. coli HB 101 déposée à la CNCM sous le N°. I-306.

Le plasmides suivants: pHS 3188 (7,6 kb), pHS 4011 (11,5 kb) et pHS 4033 (17 kb) ont été obtenus.

Les mêmes conclusions ont été atteintes par une autre approche, laquelle a consisté à cloner sur le cosmide pJB8 les séquences plasmidiques nécessaires à la pénétration intracellulaire. Ces séquences résidaient sur des insérats d'environ 45 kb, obtenus à partir d'un plasmide pWR 100.

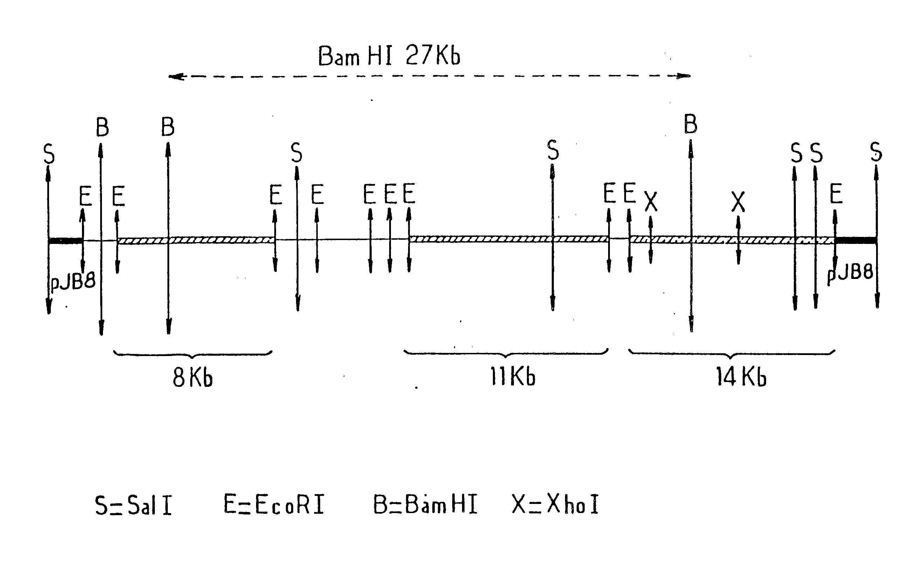

En particulier, à partir de l'un des clones invasifs pHS 4108 ainsi obtenus, il a été isolé un fragment de 45 kb originaire du plasmide pWR 100 et reconnu par son hybridation avec un fragment BamHI de 27 kb tel que défini plus haut. La carte de restriction de ce fragment de 45 kb est indiquée dans la figure 1. On y a également indiqué la position relative du fragment de BamHI de 27 kb utilisé comme sonde en sus des différents sites de restriction qui ont été reconnus.

La figure unique fait également apparaître de part et d'autre du fragment de 45 kb des parties du cosmide pJB8 initialement mis en oeuvre (en traits épais noirs) et trois fragments EcoRI de 7,6 kb, 11,5, kb et 14 kb (respectivement représentés par des doubles traits hachurés) qui se sont avérés hybrider respectivement avec les fragments de 7,6 kb, 11,5 kb et 17 kb identifiés par la première méthode.

Les différents sites de restriction sont dans la figure unique désignés par les enzymes de restriction qui leur correspondent. On remarquera que le fragment de 14 kb correspond pour l'essentiel au fragment de 17 kb isolé par la première méthode.

Les trois fragments EcoRI ainsi identifiés et isolés ont, comme le fragment BamHI de 27 kb, été testés pour leur capacité d'hybridation sélective avec les acides nucléiques isolés à partir de centaines de souches virulentes de shigelles et d'*E. coli* entéro-invasifs. Au contraire ils se sont avérés ne présenter aucune capacité d'hybridation avec des shigelles ou *Escherichia coli* entéro-invasifs inactivés par perte du plasmide de virulence ou délétion de celui-ci des séquences génétiques nécessaires à la pénétration intra-cellulaire. De même ces fragments sont inaptes à s'hybrider à des compositions d'acides nucléiques provenant d'autres espèces de bactéries. Des exemples de telles bactéries seront mentionnés ci-après dans des conditions dans lesquelles la sélectivité de ces divers fragments a été testée.

Il résulte donc de ce qui précède que les fragments EcoRI ayant des tailles de l'ordre de 7,6, de l'ordre de 11,5 kb et de l'ordre de 17 kb possèdent une séquence au moins partielle en commun avec celles du fragment BamHI de 27 kb qui a été défini plus haut.

Le fragment de 7,6 kb présente un intérêt particulier. Il a été constaté que des cultures de shigelles avirulentes transformées avec le plasmide pHS 3188 (contenant le fragment de 7,6 kb) exprimait une protéine de surface ayant une masse moléculaire de l'ordre de 23.000 Dal. Cette protéine n'était plus exprimée lorsque l'on utilisait pour la transformer le plasmide pHS 3188 contenant le fragment EcoRI de 7,6 kb dans lequel avait été inséré Tn5. Cette protéine s'est avérée être semblable à la protéine d'environ 23 000 Dal qui peut être détectée à la surface de souches virulentes de shigelles, protéine dont l'expression est cependant réprimée lorsque le souche est inactivée par culture à 30°C.

La séquence EcoRI d'environ 7,6 kb apparaît donc comme particulièrement critique pour l'expression de la virulence. Elle contient un ou plusieurs gènes essentiels à la manifestation du pouvoir invasif des bactéries virulentes. Les fragments EcoRI qui peuvent aisément être isolés à partir d'un fragment plus important issue de pWR 100 et capables de s'hybrider avec le fragment BamHI de 27 kb, sont d'un intérêt particulier, non seulement en raison de leur sélectivité, mais également de leur tailles plus réduites, qui permettent de les cloner dans des plasmides courants comme dans le pBR 325, donc de les amplifier.

La grande sélectivité de ces divers fragments conformes à l'invention a été établie en opérant dans les conditions qui seront décrités ci-après.

Préparation et marquage de la sonde d'ADN

Le plasmide de virulence a été extrait d'une culture de *S. flexneri* souche M 90 T comme décrit dans l'article de Sansonetti et coll., dans l'article des Ann Microbiol. de l'Institut Pasteur, 134A: 295—318 (1983). La purification finale a été réalisée dans un gradient de chlorure de césium. Des fragments de restriction Bam HI du plasmide de virulence ont ensuite été obtenus par traitement de ce dernier avec l'enzyme Bam HI, selon les indications du fabricant qui le commercialise (Boehringer, Mannheim, Allemagne). Ces fragments ont été séparés par électrophorèse dans un gel vertical d'agarose à 0,7%, fondant à température basse (commercialisé par la société B.R.L. de Gaithersburg, M. D. (U.S.A.), à 40 volts pendant 16 heures. Le fragment Bam HI de 27 kb, contenant les séquences nécessaires pour la pénétration intracellulaire est alors récupéré à partir de la bande de gel le contenant, par fusion à 65°C dissolution dans un faible volume du tampon E (TRIS 0,05 M; EDTA 0,02 M, pH 8,0). L'ADN a ensuite été extrait par le phénol, précipité par l'acétate de sodium et l'alcool à −20°C, centrifugé et remis en suspension dans le tampon E. 500 ng environ de l'ADN ainsi obtenu ont été marqués radio-activement par la technique dite de "Nick translation" avec des nucléotides marquées par le $^{32}P$ (Amersham International) selon la technique décrite par Rigby et coll. (1977) J. Mol. Biol. 113: 237—251. Une sonde ayant une activité de $6 \times 10^6$ à $10 \times 10^6$ cpm/µg d'ADN a ainsi été obtenue.

Cette sonde a été utilisée pour détecter des shigelles ou des *E. coli* entéro-invasifs dans les conditions qui seront décrites plus loin. Elles ont également été utilisées comme sondes pour repérer et isoler des fragments *EcoRI* ayant des poids moléculaires de l'ordre de 7,6 kb, 11,5 kb et 17 kb, respectivement obtenus par traitement du même plasmide de virulence par l'enzyme de restriction EcoRI, dans des conditions sensiblement analogues à celles qui ont été indiquées ci-dessus. Ces fragments EcoRI ont également été marqués radio-activement dans des conditions semblables.

## Préparation des échantillons de selles

Des selles obtenues à partir de patients atteints de dysentérie et de témoins ont été traitées en vue de leur dépôt sur des filtres de nitrocellulose pour des essais d'hybridation. Ces selles ont été suspendues dans un volume approprié d'eau distillée. Les dilutions obtenues ont été déposées sur des filtres de nitrocellulose préparés comme décrit ci-après.

## Préparation des filtres de nitro-cellulose

Des bactéries ont été mises en culture durant la nuit à 37°C dans de l'eau peptonée (contenant 5 g/l de NaCl, 20 g/l de peptone exempte d'indole). Des petites quantités de la culture ou des selles ont été déposées sur un filtre de nitrocellulose (BA 85, Schleicher Schuell. Dassel, Allemagne) lui-même placé à la surface d'une plaque d'agar de Mac-Conkey. 20 différentes souches ont été déposées sur des filtres de la même manière. Après croissance à 37°C, les bactéries ont été lysées de l'ADN a été dénaturé comme décrit par Moseley et coll. (1980) J. Infect. dis. (1980) 142: 892—898. Les filtres ont été placés pendant 10 minutes sur du papier Whatman No. 3 saturé par de la soude 0,5 M. Quatre transferts d'une durée de 1 minute chacun ont ensuite été réalisés sur papier saturé avec de l'acétate d'ammonium 0,1 M et de la soude 0,02 M. Les filtres ont été écartés puis séchés à l'air soigneusement, après un 5 ème transfert pendant 10 minutes sur un papier saturé avec de l'acétate d'ammonium-soude, avant d'être soumis à cuisson pendant la nuit à 65°C. Les filtres ainsi traités ont été conservés à la température ambiante.

## Hybridation sur les filtres

Les filtres ont été incubés à 42°C dans une solution de pré-hybridation (formamide 50%, 5×SSC (1×SSC:NaCl 0,15 M; citrate de sodium 0,015 M), 5×solution de Denhardt, ADN de thymus de veau dénaturé par la chaleur 100 µg/ml). Les filtres ont ensuite été placés dans des sacs de plastique scellés contenant 10 ml de la solution d'hybridation (formamide 50%, 5×SSC, 1×solution de Denhardt, sulfate de dextrane à 10%, phosphate de sodium 0,02 M pH 6,5) 100 µg/ml d'ADN de thymus de veau et $10^6$ cpm de la sonde d'ADN, les deux ADN ayant au préalable été dénaturés par la chaleur). On a laissé l'hybridation se produite pendant la nuit à 42°C. Les filtres ont ensuite été lavés comme suit: trois lavages pendant 5 minutes dans 2×SSC et SDS 0,1%, puis deux fois pendant 30 minutes à 50°C dans 0,1×SSC et SDS 0,1%. Les filtres ont ensuite été séchés à l'air avant d'être exposés pendant 6 heures à −70°C à un film Kodak X-OMAT[R] placé entre deux écrans d'intensification (Philips France). Le film a ensuite été développé selon les instructions du fabricant.

Les différentes sondes selon l'invention ont été utilisées sur des centaines de shigelles et de *E. coli* entéro-invasifs. Des essais témoins ont été réalisés avec extraits d'Entériobacteriaceae, parmi lesquelles des bactéries entéro-invasives telle que *Salmonellae* et *Yersiniae.* D'autres bactéries gram-négatives ont également été testées, parmi lesquelles des agents causes de diarrhées tels que des *Vibrios* et des *Plesiomonas.*

Des essais ont également été réalisés avec des témoins de shigelles et de *E. coli* entéro-invasifs ayant perdu leurs plasmides de virulence. Les essais ont été effectués sur plusieurs types de shigelles, notamment *S. sonnei, S. flexneri, S. boydii* et *S. dysenteriae.* Les essais ont porté sur plus de 300 isolats de shigelles.

Les résultats des essais d'hybridation ont montré que la sensibilité de la méthode était de 99,7% et la spécificité de 100%. Tout au plus observe-ton certaines variations au niveau de l'intensité des autoradiogrammes obtenus avec des colonies virulentes de *S. sonnei*, peut-être en raison de l'instabilité élevée, connue, de leurs plasmides de virulence. On n'a obtenu pratiquement aucune hybridation avec les ADN des bactéries témoins (bactéries d'espèces différentes ou shigelles ou E. coli entéroinvasifs, dont les plasmides de virulence étaient, soit perdus, soit pourvus de délétions inactivantes).

Des essais quantitatifs selon les teneurs en bactéries des dilutions initialement déposées sur les filtres de nitrocellulose) ont montré que les sondes selon l'invention pouvaient détecter $10^3$ unités formant colonies (C.F.U.) de shigelles, soit des dilutions de 10 à 100 fois plus élevées que les dilutions maxima détectées par les procédures bactériologiques antérieures.

Des essais ont été faits sur des échantillons de selles diarrhéiques. Les résultats d'hybridation avec les 3 types de sondes (7,6; 11,5 et 17 kb) selon l'invention ont permit de confirmer la plus grande sensibilité de la sonde 17 kb pour le diagnostic des shigelles.

Dans ce qui précède il a été question de sondes marquées radio-activement. Bien entendu l'invention ne se limite pas à ce mode de marquage. De façon en soi connue, les sondes peuvent être modifiées par un group chimique permettant leur couplage, direct ou indirect, par exemple avec une enzyme dont la présence peut être révélée par son action à l'égard d'un substrat spécifique, de préférence un substrat chromogène, ou encore avec une molécule fluorescente ou luminescente. Des modes de couplage de ce type ont été envisagés par exemple dans les brevets français 78 10975 et 81 24631 ou dans la demande de brevet européen publiée 0063879.

L'invention concerne naturellement également le procédé de détection lui-même des shigelles et *E. coli* entéro-invasifs virulents mettant en oeuvre les sondes selon l'invention. D'une façon générale les principales étapes du procédé de détection selon l'invention comprennent:

—le dépôt et la fixation des acides nucléiques des cellules à analyser (ou de ces dernières préalablement traitées de façon à permettre l'accès de leurs acides nucléiques à la sonde) sur un filtre approprié, tel que filtre de nitrocellulose ou analogue;

—la mise en contact des acides nucléiques ainsi fixés ou rendus accessibles avec la sonde marquée selon l'invention dans des conditions permettant la réalisation d'une hybridation effective, lorsque les bactéries recherchées sont présentes;

—l'élimination, notamment par lavage, de toute sonde non hybridée et

—la détection des sondes d'ADN retenues par hybridation sur le support.

L'invention concerne naturellement également les nécessaires ou "kits" contenant une sonde conforme à l'invention et le cas échéant des préparations témoins de selles, ou des préparations témoins d'acides nucléiques provenant de souches avirulentes et tous autres réactifs nécessaires au dosage.

L'invention n'est cependant pas limitée à l'application des sondes selon l'invention à des épreuves de diagnostic *in vivo* de shigelloses. Elle peut également être mise en eouvre pour le dépistage systématique de shigelles ou de E. coli entéro-invasifs dans l'environnement normal de l'homme, par exemple dans l'eau, les aliments, etc. Elle peut également être utilisée à des fins d'analyse, notamment de classification de bacéries virulentes nouvellement isolées, selon que les acides nucléiques de ces dernières peuvent donner lieu à hybridation ou non avec les sondes selon l'invention. Elle peut également être utilisée pour l'isolement de séquences complémentaires à partie d'acides nucléiques préalablement fragmentés, dans des opérations d'hybridation sélective. Dans une telle hypothèse les séquences d'acides nucléiques selon l'invention sont de préférence fixés de façon en soi connue sur un support insoluble. L'opération de séparation comprendra alors la mise en contact de la sonde fixée sur le support insoluble avec la solution d'acide nucléique présumée contenir les fragments à séparer dans des conditions susceptibles d'autoriser l'hybridation, la séparation des acides nucléiques non fixés et la récupération des fragments retenus, après dénaturation des hybrides préalablement formées.

Enfin les fragments d'ADN selon l'invention, et plus particulièrement le fragment EcoRI de 7,6 kb environ peut être utilisé dans des études visant à la production de vaccins efficaces contre les shigelles, notamment sous réserve de sont incorporation stable dans un chromosome de *E. coli* atoxique, et ce dans des conditions permettant l'expression ultérieure par *E. coli* de ce fragment, notamment sous la forme d'une protéine ayant les propriétés de la protéine de surface de 23.000 daltons dont il a été question plus haut.

Comme il va de soi et comme il résulte d'ailleurs déjà de ce qui précède, l'invention ne se limite nullement à ceux de ses modes d'application et de réalisation qui ont été spécialement envisagés; elle en embrasse, ou contraire, toutes les variantes.

En particulier il va de soi que les définitions contenues dans les revendications doivent être considérées comme ayant la portée des définitions envisagées dans la description. Les revendications étendent bien entendu leurs effets également, tout particulièrement à toute sonde hybridant avec les séquences préférées qui ont été définies dans la présente demande de brevet, mais n'hybridant pas avec les séquences nucléiques susceptibles d'être obtenues à partir de shigelles ou de E. coli entéro-invasifs inactivés ou dépourvus de virulence. Il s'agit en particulier des bactéries contenant encore des parties du plasmide de virulence, dont ont cependant été délétés les séquences intra-géniques auxquelles peut être attribuée ladite virulence.

## Revendications

1. Sonde pour la détection des shigelles et des *E. coli* entéro-invasifs, comprenant une séquence d'acide nucléique originaire du plasmide de virulence de 140 Mdal de la souche M 90 T *Shigella flexneri* déposée à la C.N.C.M. sous le N° I-308, ayant une taille au plus égale à environ 27 kb et comprenant la totalité ou une partie du fragment Bam HI de 27 kb.

2. Sonde selon la revendication 1, caractérisée en ce que ladite séquence est constituée par une séquence EcoRI ayant une taille de l'ordre de 7,6 kb.

3. Sonde selon la revendication 1, caractérisée en ce que ladite séquence est constituée par une séquence EcoRI ayant une taille de l'ordre de 11,5 kb.

4. Sonde selon la revendication 1, caractérisée en ce que ladite séquence est constituée par une séquence EcoRI ayant une taille de l'ordre de 17 kb.

5. Sonde selon l'une quelconque des revendications 2 à 4, caractérisée en ce qu'elle est insérée à l'intérieur d'un plasmide, tel que pBR 325.

6. Nécessaire pour la détection ou le diagnostic des shigelles et *E. coli* entéro-invasifs virulents, qui comporte une sonde conforme à l'une quelconque des revendications 1 à 5 et des préparations témoins d'acides nucléiques provenant de souches avirulentes ou de selles provenant d'individus témoins sains.

7. Procédé de détection, notamment de diagnostic *in vitro*, de la présence de shigelles et de *E. coli* entéro-invasifs virulents, caractérisé en ce qu'il met en oeuvre une sonde conforme à l'une quelconque des revendications 1 à 5, ce procédé comprenant:

—le dépôt et la fixation des acides nucléiques des cellules à analyser (ou de ces dernières préalablement traitées de façon à permettre l'accès de leurs acides nucléiques à la sonde) sur un filtre approprié, tel que filtre de nitro-cellulose ou analogue;

—la mise en contact des acides nucléiques ainsi fixés ou rendus accessibles avec la sonde marquée selon l'invention dans des conditions permettant la réalisation d'une hybridation effective, lorsque les bactéries recherchées sont présentes;

—l'élimination, notamment par lavage, de toute sonde non hybridée et

—la détection des sondes d'ADN retenues par hybridation sur le support.

## Patentansprüche

1. Sonde zum Nachweis von Shigellen und entero-invasiven *E. coli* enthaltend eine Nucleinsäuresequenz aus dem Virulenz-Plasmid von 140 Mdal des Stammes M90T *Shigella flexneri*, der beim C.N.C.M. unter der Hinterlegungsnummer I-308 hinterlegt worden ist, mit einer Größe von höchstens etwa 27 kb, welche das gesamte Fragment Bam HI von 27 kb oder einen Teil davon umfaßt.

2. Sonde nach Anspruch 1, dadurch gekennzeichnet, daß die Sequenz eine Eco-RI-Sequenz mit einer Größe von etwa 7,6 kb ist.

3. Sonde nach Anspruch 1, dadurch gekennzeichnet, daß die Sequenz eine Eco-RI-Sequenz mit einer Größe von etwa 11,5 kb ist.

4. Sonde nach Anspruch 1, dadurch gekennzeichnet, daß die Sequenz eine Eco-RI-Sequenz mit einer Größe von etwa 17 kb ist.

5. Sonde nach einem der Ansprüche 2 bis 4, dadurch gekennzeichnet, daß sie in das Innere eines Plasmids, wie pBR 325, eingebracht ist.

6. Kit zum Nachweisoder zur Diagnose von virulenten Shigellen und entero-invasiven *E. coli*, umfassend eine Sonde nach einem der Ansprüche 1 bis 5 und Kontrollpräparate von Nucleinsäuren, die von avirulenten Stämmen oder dem Stuhl von gesunden Kontrollindividuen stammen.

7. Verfahren zum Nachweise und insbesonder zur *in vitro*-Diagnose der Anwesenheit von virulenten Shigellen und entero-invasiven *E. coli*, dadurch gekennzeichnet, daß man eine Sonde nach einem der Ansprüche 1 bis 5 einsetzt, welches Verfahren die folgenden Maßnahmen umfaßt:

—die Abscheidung und Fixierung der Nucleinsäuren der zu analysierenden Zellen (oder der zu analysierenden Zellen, die zuvor derart behandelt worden sind, daß ihre Nucleinsäuren der Sonde zugänglich sind) auf einem geeigneten Filter, wie einem Nitro-Zellulosefilter oder dergleichen;

—in Kontakt bringen der in dieser Weise fixierten oder zugänglich gemachten Nucleinsäuren mit der markierten erfindungsgemäßen Sonde unter Bedingungen, welche eine effektive Hybridisierung ermöglichen dann, wenn die gesuchten Bakterien vorhanden sind;

—Entfernung der nichthybridisierten Sonde, insbesondere durch Waschen und

—Nachweis der DNS-Sonden, die durch Hybridisierung auf dem Träger zurückgehalten worden sind.

## Claims

1. A probe for the detection of shigellae and entero-invasive *E. coli*, comprising a nucleic acid sequence originating from the 140 Mdal virulence plasmid of the M 90 T strain of *Shigella flexneri*, deposited with the CNCM under No. I 308 having a maximum size of around 27 kb and comprising all or a part of the 27 kb Bam HI fragment.

2. The probe according to Claim 1, characterised in that said sequence is formed with an EcoRI sequence having a size of the order of 7.6 kb.

3. The probe according to Claim 1, characterised in that said sequence is formed with an EcoRI sequence having a size of the order of 11.5 kb.

4. The probe according to Claim 1, characterised in that said sequence is formed with an EcoRI sequence having a size of the order of 17 kb.

5. The probe according to any of the Claims 2 to 4, characterised in that it is inserted into a plasmid, such as pBR 325.

6. A kit for the detection or the diagnosis of virulent shigellae and entero-invasive *E. coli*, which includes a probe conforming to any of the Claims 1 to 5 and the control preparations of nucleic acids from avirulent strains or of stools from healthy control individuals.

7. A process of detection, notably of *in vitro* diagnosis, of the presence of virulent shigellae and entero-invasive *E. coli*, characterised in that it uses a probe conforming to any of the Claims 1 to 5, this process including:

—the deposition and fixation of the nucleic acids of the cells to be analyzed (or the cells themselves) previously treated in such a manner as to permit access of their nucleic acids to the probe) onto an appropriate filter, such as one of nitrocellulose or analogue;

—the bringing into contact of the nucleic acids thus fixed or rendered accessible, with the labelled probe according to the invention under conditions permitting effective hybridization, when the bacteria being sought are present;

—the elimination, notably by washing, of any non-hybridization probe; and

—the detection of the probes for DNA retained by hybridization on the support.

BamHI 27Kb

pJB8

S — E — B — B — E — E — S — E — E — E — E — S — E — X — B — X — S — S — E — S

8Kb    11Kb    14Kb

pJB8

S=Sal I   E=EcoRI   B=BamHI   X=XhoI

1